# EUROPEAN PATENT APPLICATION

(11) **EP 1 867 329 A2**
(43) Date of publication of application: **19.12.2007**
(21) Application number: 07110169.5
(22) Date of filing: 13.06.2007
(51) Int. Cl.: A61K 31/33, A61K 31/44, A61P 25/28, A61P 31/18, A61P 33/06, A61P 43/00

(54) **5-Amino-4-hydroxy-2-isopropyl-7-[4-methoxy-3-(3-methoxypropoxy)benzyl]-8-methylnonanamides as therapeutic compounds**

(30) Priority: 14.06.2006 CH 9722006
(71) Applicant: Speedel Experimenta AG, 4123 Allschwil (CH)
(72) Inventor: Herold, Peter, 4123, Allschwil (CH); Mah, Robert, 4123, Allschwil (CH); Stutz, Stefan, 4123, Allschwil (CH); Tschinke, Vincenzo, 4123, Allschwil (CH); Schumacher, Christoph, 4123, Allschwil (CH)
(74) Representative: Maué, Paul Georg

(57) **Abstract**

Use of compounds of the general formula (1) 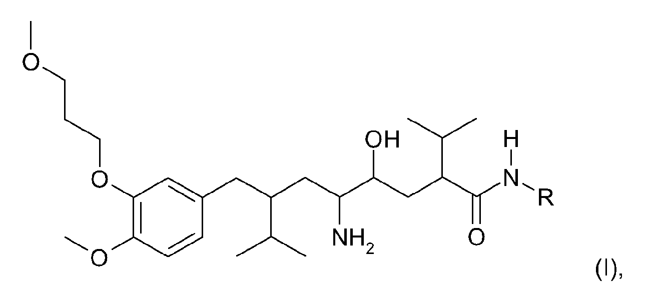 and pharmaceutically usable salt thereof, in which the substituent R has the definitions illustrated in detail in the description, as beta-secretase, cathepsin D, plasmepsin II and/or HIV protease inhibitors.

## Description

### Field of the Invention

The present invention relates to the use of 5-amino-4-hydroxy-2-isopropyl-7-[4-methoxy-3-(3-methoxypropoxy)benzyl]-8-methylnonanamides as beta-secretase-, cathepsin D-, plasmepsin II- and/or HIV-protease-inhibitors.

### Background of the Invention

With regard to beta-secretase-, cathepsin D-, plasmepsin II- and/or HIV-protease-inhibition, there is still a need for highly potent active ingredients. In this context, the improvement of the pharmacokinetic properties is at the forefront. These properties directed towards better bioavailability are, for example, absorption, metabolic stability, solubility or lipophilicity.

### Alzheimer Disease aspartyl protease: Beta-Secretase

Alzheimer's disease (AD) is a progressive degenerative disease of the brain. The symptoms of AD include progressive memory loss, language difficulty and ultimately loss of basic neural function and death. The biomarkers in the central nervous system for AD include amyloid plaques, intracellular neurofibrillary tangles and activated microglia. The appearance of these three markers is likely to contribute to the neuronal cell death and memory loss observed in AD.

Beta-amyloid is a defining feature of AD and now believed to be a causative precursor in the development of the disease. Amyloidogenic plaques and vascular amyloid angiopathy also characterize the brains of individuals with Trisomy 21 (Down's Syndrome), Hereditary Cerebral Hemorrhage with Amloidosis of the Dutch-Type (HCHWA-D) and other neurodegenerative disorders.

Beta-amyloid plaques are predominantly composed of amyloid beta peptide (A-beta, also sometimes designated betaA4). The A-beta peptide is derived by proteolysis of the beta amyloid precursor protein (APP). Beta-APP is processed by three distinct ordered enzymatic activities. The bulk of beta-APP is processed via alpha-secretase in a non-amyloidogenic pathway. A small fraction of beta-APP is cleaved by beta-secretase activity to generate the membrane-bound Carboxy-terminal fragment C99. Gamma-secretase cleaves C99 to generate the amyloidogenic A-beta peptide of 39-42 amino acids. The aspartyl protease activity of beta-secretase has been disclosed using varied nomenclature, including BACE (beta-site APP cleaving enzyme), Asp and memapsin.

The significance of beta-secretase cleavage of beta-APP as a critical step in the generation of AD is underscored by the observation that human mutations at the beta-secretase cleavage subsites (Swedish mutations) of beta-APP lead to increased A-beta production and early onset familial AD. Furthermore, BACE1- knockout mice fail to produce A-beta peptide and present a normal phenotype. When crossed with transgenic mice that overexpress APP, the progeny show reduced amounts of A-beta in brain extracts as compared with control animals. This evidence supports the proposal that inhibition of beta-secretase activity and reduction of A-beta peptide deposits in the brain provides a therapeutic strategy for the treatment of AD and other beta amyloid disorders as described by Verdile et al. (2004) in Pharmacol. Res 50, 397-409.

Compounds that are effective inhibitors of beta-secretase may inhibit beta-secretase-mediated cleavage of APP and the production of A-beta peptide. The pharmacological inhibition of A-beta peptide generation may reduce amyloid beta deposits, respectively the formation of plaques. Beta-secretase inhibiting compounds as discussed by Thompson et al. (2005) in Curr. Pharm. Des. 11, 3383-3404 are therefore useful to treat or to prevent diseases that are characterized by amyloid beta deposits or plaques such as AD.

The present invention also relates to methods of treating subjects who have, or in preventing subjects from developing a disease or condition selected from the group consisting of AD, for helping prevent or delay the onset of AD, for helping to slow the progression of AD, for treating subjects with mild cognitive impairment (MCI) and preventing or delaying the onset of AD in those who could progress form MCI to AD, for treating Down's syndrome, for treating humans who have HCHWAD, for treating cerebral amyloid angiopathy, and for treating degenerative dementias.

### Alzheimer's Disease aspartyl protease: Cathepsin D

Human cathepsin D is an intracellular aspartic peptidase found mainly in lysosomes. It has a number of housekeeping functions, including the degradation of cellular and phagocytosed proteins. The enzymes may be involved in a variety of disease states, including cancer and Alzheimer's disease (AD). Clinical studies have shown that cathepsin D is overexpressed in breast cancer cells and this seems to be associated with an increased risk for metastasis due to enhanced cell growth. Cathepisn D is also thought to be involved in formation of the beta-amyloid peptide in AD. Recently, several genetic association studies linked cathepsin D with amyloid pathology and Alzheimer's disease as described for example by Davidson et al., (2006) in J. Neurol. Neurosurg. Psychiatry 77, 515-517. The availability of selective and potent inhibitors will help to further define the role of cathepsin D in disease and possibly lead to therapeutic agents.

### Malaria Aspartyl Protease: Plasmepsin I and II

Malaria is considered as one of the most serious infectious diseases in the world, affecting approximately 500 million people. The disease is spread by the anopheles mosquito that is mostly found in tropical regions. The species plasmodium falciparum is responsible for more than 95% of malaria-related morbidity and mortality. Increasingly, plasmodium falciparum is becoming resistant to existing therapies such as chloroquine, mefloquine and sulfadoxime/ pyrimethamine. Thus there is an urgent need for new treatments.

In the erythrocytic stage of the parasite's life cycle the parasite invades the red blood cells of its host consuming up to 80% of the hemoglobin as a source of nutrients for growth and development. Hemoglobin degradation takes place in an acidic vacuole of the parasite and many of the current antimalarial drugs appear to disrupt important vacuolar functions. The food vacuole contains aspartic, cysteine and metallo-proteases, which are all considered to play a role in the process of hemoglobin degradation. At least 10 genes encoding aspartic proteases have been identified in the plasmodium genome. Four of the aspartic proteases have been localized in the acidic food vacuole of the parasite, namely plasmepsin I, II, IV and HAP, a histoaspartic protease. Inhibitors of plasmepsin I and II have shown efficacy in cell and animal models of malaria, indicating that these enzymes may represent targets for drug discovery as described for example by Coombs et al. (2001) Trends Parasitol 17, 532-537. Indeed, a non-selective inhibitor of aspartic proteases, pepstatin, inhibits the growth of plasmodium falciparum in vitro. Similar results have been obtained with analogs of pepstatin or with immunodeficiency virus protease inhibitors indicating that inhibition of aspartic proteases interferes with the life cycle of plasmodium falciparum as noted for example by Andrews et al. (2006) in Antimicrob. Agents Chemother 50, 639-648.

The present invention relates to the identification of low molecular weight, nonpeptidic inhibitors of the plasmodium falciparum protease plasmepsin II or other related aspartic proteases to treat and/or to prevent malaria.

### HIV aspartyl protease: HIV-1 peptidase

First reported in 1981 in a small number of patients, Acquired immunodeficiency syndrome (AIDS) has now become a major epidemic with more than 38 million people infected worldwide, including approximately 1 million in the United States, 580,000 in Western Europe and more than 25 million in Sub-Saharan Africa (http://www.unaids.org). Since AIDS was first clinically identified, scientific and therapeutic progress has been extraordinary. However, AIDS remains out of control, especially in developing countries.

The prognosis of AIDS patients who have full access to current therapies has completely changed since the first cases of AIDS were reported. Today, the median survival for HIV-positive patients receiving treatment exceeds 8 years. The life expectancy for AIDS patients was less than 1 year before AZT was introduced in 1987. This dramatic change is due to the development of effective therapies, to early detection of HIV-positive individuals, and to a sustained effort to analyze and understand viral-resistance mechanisms, which can be overcome by rational drug development and combination therapy.

FDA-approved therapies target three steps of the HIV life cycle: reverse transcription, proteolytic maturation and fusion. Triple therapy, commonly referred to as HIGHLY ACTIVE ANTIRETROVIRAL THERAPY (HAART), is now the standard for treatment. It consists of a protease inhibitor or a non-nucleoside reverse transcriptase inhibitor in combination with two nucleoside reverse transcriptase inhibitors.

Translation of human immunodeficiency virus type-1 (HIV-1) genomic RNA results in the production of two polyprotein precursors, Gag and Gag-Pol. The 55-kDa Gag precursor contains the structural proteins and the 160-kDa Gag-Pol polyprotein contains the functional viral enzymes protease, reverse transcriptase, and integrase. Gag and Gag-Pol polyproteins are transported to the plasma membrane where assembly of type-C retroviruses and lentiviruses typically occurs. During particle assembly, the viral protease cleaves the Gag and Gag-Pol precursors into the structural and functional proteins required for viral replication. The protease activity within the cytoplasma of infected cells allows for the formation of virions which can be released from the cell in the last stages of budding.

The mature HIV-1 protease is an obligatory dimer of identical 11-kDa subunits, each contributing one of the two catalytic aspartic residues. In contrast, the cell-derived members of the aspartic protease family are monomeric enzymes with two Asp-Thr-Gly-containing domains. The unique dimeric structure of the retroviral protease is mainly stabilized by an antiparallel beta-sheet formed by the interdigitation of the amino- and carboxyl-terminal beta-strands of each monomer.

The activation of HIV-1 protease i.e. the dimerization and autocatalytic release from Gag-Pol, is a critical step in the viral life cycle. Inhibition of protease activation causes a severe defect in Gag polyprotein processing and a complete loss of viral infectivity. As such, the viral protease has become a target for HIV therapeutics, resulting in many HIV protease inhibitors reaching clinical trials as reviewed by Rana et al. (1999) in Pharmacotherapy 19, 35-59 and Morse et al., (2006) in Lancet Infect. Dis. 6, 215-225. Most of these drugs are substrate-based inhibitors, whose design has been facilitated by an abundance of crystal structure data for both the native enzyme and enzyme-inhibitor complexes. Additionally, there are now extensive biochemical data existing, detailing both the catalytic mechanism and the molecular basis for substrate selection.

### Detailed Description of the Invention

Firstly, the present invention relates to the use as beta-secretase-, cathepsin D-, plasmepsin II- and/or HIV-protease-inhibitors of compounds of the general formula (I): 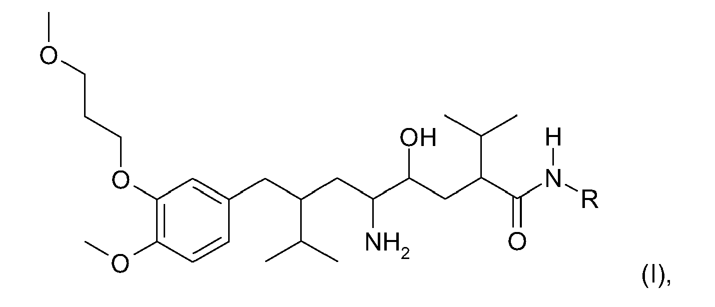 in which
R is heterocyclyl which is bonded via a carbon atom and is mono- or polysubstituted by C₁₋₆-alkyl, trifluoromethyl, nitro, amino, C₁₋₆-alkylamino, di-C₁₋₆-alkylamino, C₂₋₆-alkenyl, C₂₋₆-alkynyl, C₁₋₆-alkoxy, C₁₋₆-alkylcarbonyloxy, C₀₋₆-alkylcarbonylamino, C₁₋₆-alkoxycarbonylamino, hydroxyl, halogen, oxide, cyano, carbamoyl or N-mono- or N, N-di-C₁₋₈-alkylated carbamoyl, carboxyl or esterified carboxyl or C₁₋₆-alkylenedioxy, or is substituted by oxo and mono- or polysubstituted by C₁₋₆-alkyl, trifluoromethyl, nitro, amino, C₁₋₆-alkylamino, di-C₁₋₆-alkylamino, C₂₋₆-alkenyl, C₂₋₆-alkynyl, C₁₋₆-alkoxy, C₁₋₆-alkylcarbonyloxy, C₀₋₆-alkylcarbonylamino, C₁₋₆-alkoxycarbonylamino, hydroxyl, halogen, oxide, cyano, carbamoyl or N-mono- or N,N-di-C₁₋₈-alkylated carbamoyl, carboxyl or esterified carboxyl or C₁₋₆-alkylenedioxy, and salts, especially pharmaceutically usable salts thereof.

The term heterocyclyl denotes 4-16-membered, mono- or bicyclic, saturated and unsaturated heterocyclic radicals having 1 to 4 nitrogen and/or 1 or 2 sulphur or oxygen atoms which are substituted. Preference is given to 4-8-membered, particular preference to 5-6-membered, monocyclic radicals which optionally have a 4-8-membered fused-on ring which may be carbocyclic or heterocyclic, and is more preferably a fused-on benzo or pyrido ring. Preferred heterocyclic radicals have, per ring, 1-4 nitrogen atoms, 1-2 oxygen or sulphur atoms, 1-2 nitrogen atoms and 1-2 oxygen atoms, or 1-2 nitrogen atoms and 1-2 sulphur atoms, with at least one carbon atom, preferably 1-7 carbon atoms, being present per ring. Examples of heterocyclyl radicals are pyridyl, thienyl, pyrazinyl, triazolyl, imidazolyl, benzothiazolyl, furyl, pyrimidinyl, quinazolinyl, quinolyl, quinoxalinyl, isoquinolyl, benzo[b]thienyl, isobenzofuranyl, benzimidazolyl, oxazolyl, thiazolyl, indolyl, pyrrolyl, piperidinyl, pyrrolidinyl, pyranyl, tetrahydropyranyl, tetrahydrofuranyl, 1H-pyrrolizinyl, phthalazinyl, [1,5]naphthyridyl, dihydro-2H-benzo[1,4]thiazinyl, dihydro-1 H-pyrido[2,3-b][1,4]oxazinyl, 1H-pyrrolo[2,3-b]pyridyl, benzo[1,3]dioxolyl, benzooxazolyl, 3,4-dihydrobenzooxazinyl, 2,3-dihydroindolyl, indazolyl or benzofuranyl.

C₁₋₆-Alkyl may be straight-chain or branched and/or bridged and is, for example, methyl, ethyl, propyl, isopropyl, butyl, isobutyl, sec-butyl, tert-butyl or a pentyl or hexyl group.
C₁₋₆-Alkylamino is, for example, methylamino, ethylamino, propylamino or butylamino.
Di-C₁₋₆-alkylamino is, for example, dimethylamino, N-methyl-N-ethylamino, diethylamino, N-methyl-N-propylamino or N-butyl-N-methylamino.
C₂₋₆-Alkenyl may be straight-chain or branched and is, for example, allyl or vinyl.
C₂₋₆-Alkynyl may be straight-chain or branched and is, for example ethynyl.
C₁₋₆-Alkoxy is, for example, methoxy, ethoxy, propyloxy, isopropyloxy, butyloxy, isobutyloxy, sec-butyloxy, tert-butyloxy, pentyloxy or hexyloxy.
C₁₋₆-Alkoxycarbonylamino is preferably C₂₋₅-alkoxycarbonylamino such as ethoxycarbonylamino, propyloxycarbonylamino, isopropyloxycarbonylamino, butyloxycarbonylamino, isobutyloxycarbonylamino, sec-butyloxycarbonylamino or tert-butyloxycarbonylamino.
C₁₋₆-Alkylcarbonyloxy is, for example, acetyloxy, propionyloxy, propylcarbonyloxy, isopropylcarbonyloxy, butylcarbonyloxy, isobutylcarbonyloxy, sec-butylcarbonyloxy, tert-butylcarbonyloxy, pentylcarbonyloxy or hexylcarbonyloxy.

C₀₋₆-Alkylcarbonylamino is, for example, formylamino, acetylamino, propionylamino, propylcarbonylamino, isopropylcarbonylamino, butylcarbonylamino, isobutylcarbonylamino, sec-butylcarbonylamino, tert-butylcarbonylamino, pentylcarbonylamino or hexylcarbonylamino.
Halogen is, for example, fluorine, chlorine, bromine or iodine, preferably fluorine or chlorine.
C₁₋₆-Alkylenedioxy is, for example, methylenedioxy, ethylenedioxy, 1,3-propylenedioxy or 1,2-propylenedioxy.
Carbamoyl or N-mono- or N,N-di-C₁₋₈-alkylated carbamoyl is, for example, carbamoyl, methylcarbamoyl, ethylcarbamoyl, N,N-dimethylcarbamoyl, N,N-diethylcarbamoyl or propylcarbamoyl.
Carboxyl or esterified carboxyl is, for example, carboxyl esterified with C₀₋₆-alkyl, such as carboxyl or C₁₋₆-alkoxycarbonyl.

Depending on the presence of asymmetric carbon atoms, the inventive compounds may be present in the form of isomer mixtures, especially as racemates, or in the form of pure isomers, especially of optical antipodes. The invention encompasses all of these forms. Diastereomer mixtures, diastereomeric racemates or mixtures of diastereomeric racemates may be separated by customary methods, for example by column chromatography, thin-layer chromatography, HPLC and the like.

Salts of compounds with salt-forming groups are in particular acid addition salts, salts with bases or, if a plurality of salt-forming groups is present, optionally also mixed salts or inner salts.

Salts are primarily the pharmaceutically acceptable or non-toxic salts of compounds of the formula (I).

Such salts are formed for example by compounds of the formula (I) having an acidic group, e.g. a carboxy or sulpho group, and are for example their salts with suitable bases, such as non-toxic metal salts derived from metals of group la, Ib, IIa and IIb of the Periodic Table of the Elements, e.g. alkali metal, in particular lithium, sodium or potassium, salts, alkaline earth metal salts, for example magnesium or calcium salts, furthermore zinc salts or ammonium salts, also salts formed with organic amines such as optionally hydroxy-substituted mono-, di- or trialkylamines, especially mono-, di- or tri-lower-alkylamines, or with quaternary ammonium bases, e.g. methyl-, ethyl-, diethyl- or triethylamine, mono-, bis- or tris(2-hydroxy-lower-alkyl)amines such as ethanol-, diethanol- or triethanolamine, tris(hydroxymethyl)methylamine or 2-hydroxytertiary-butylamine, N.N-di-lower-alkyl-N-(hydroxy-lower-alkyl)amine, such as N,N-dimethyl-N-(2-hydroxyethyl)amine, or N-methyl-D-glucamine, or quaternary ammonium hydroxides such as tetrabutylammonium hydroxide. The compounds of the formula I having a basic group, e.g. an amino group, can form acid addition salts, e.g. with suitable inorganic acids, e.g. hydrohalic acid such as hydrochloric acid, hydrobromic acid, sulphuric acid with replacement of one or both protons, phosphoric acid with replacement of one or more protons, e.g. orthophosphoric acid or metaphosphoric acid, or pyrophosphoric acid with replacement of one or more protons, or with organic carboxylic, sulphonic or phosphonic acids or N-substituted sulphamic acids, e.g. acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid, isonicotinic acid, furthermore amino acids such as, for example, the α-amino acids mentioned hereinabove, and methanesulphonic acid, ethanesulphonic acid, 2-hydroxyethanesulphonic acid, ethane-1,2-disulphonic acid, benzenesulphonic acid, 4-toluenesulphonic acid, naphthalene-2-sulphonic acid, 2- or 3-phosphoglycerate, glucose 6-phosphate, N-cyclohexylsulphamic acid (to form cyclamates) or with other acidic organic compounds such as ascorbic acid. Compounds of the formula (I) having acidic and basic groups may also form inner salts.

Pharmaceutically unsuitable salts may also be used for isolation and purification.

Prodrug derivatives of the compounds described herein are derivatives thereof which on *in vivo* use liberate the original compound by a chemical or physiological process. A prodrug may for example be converted into the original compound when a physiological pH is reached or by enzymatic conversion. Possible examples of prodrug derivatives are esters of freely available carboxylic acids, S- and O-acyl derivatives of thiols, alcohols or phenols, the acyl group being defined as above. Preferred derivatives are pharmaceutically acceptable ester derivatives which are converted by solvolysis in physiological medium into the original carboxylic acid, such as, for example, lower alkyl esters, cycloalkyl esters, lower alkenyl esters, benzyl esters, mono- or disubstituted lower alkyl esters such as lower ω-(amino, mono- or dialkylamino, carboxy, lower alkoxycarbonyl) - alkyl esters or such as lower α-(alkanoyloxy, alkoxycarbonyl or dialkylaminocarbonyl) - alkyl esters; conventionally, pivaloyloxymethyl esters and similar esters are used as such.

Because of the close relationship between a free compound, a prodrug derivative and a salt compound, a particular compound in this invention also includes its prodrug derivative and salt form, where this is possible and appropriate.

The compounds of the formula (I) also include those compounds in which one or more atoms are replaced by their stable, non-radioactive isotopes; for example, a hydrogen atom by deuterium.

R is preferably a 5-10-membered mono- or bicyclic radical having at least one nitrogen or oxygen atom and optionally one oxygen or sulphur atom. Most preferably, R is pyridyl, thiazolyl, oxazolyl, indolyl, benzofuranyl, benzothiazolyl or imidazolyl.

R is preferably mono- or disubstituted. Preferred substituents are: C₁₋₆-alkyl, C₁₋₆-alkoxy, C₀₋₆-alkylcarbonylamino, C₁₋₆-alkoxycarbonylamino, trifluoromethyl, nitro, amino, hydroxyl, halogen, oxide, cyano, carbamoyl or N-mono- or N,N-di-C₁-C₈-alkylated carbamoyl and carboxyl or esterified carboxyl.

The invention preferably relates to compounds of the formula (I) in which
R is pyridyl, thiazolyl, oxazolyl, indolyl, benzofuranyl, benzothiazolyl or imidazolyl, each of which is bonded via a carbon atom and is mono- or polysubstituted by C₁₋₆-alkyl, C₀₋₆-alkylcarbonylamino, C₁₋₆-alkoxycarbonylamino, C₁₋₆-alkoxy, trifluoromethyl, nitro, amino, hydroxyl, halogen, oxide, cyano, carbamoyl or N-mono- or N,N-di-C₁-C₈-alkylated carbamoyl or carboxyl or esterified carboxyl, and their pharmaceutically usable salts.

The compounds of the formula (I) may also be prepared in optically pure form. The separation into antipodes may be effected by methods known per se, either preferably at a synthetically early stage by salt formation with an optically active acid, for example (+)- or (-)-mandelic acid and separation of the diastereomeric salts by fractional crystallization, or preferably at a rather later stage by derivatization with a chiral auxiliary building block, for example (+)- or (-)-camphanoyl chloride, and separation of the diastereomeric products by chromatography and/or crystallization and subsequent cleavage of the bond to the chiral auxiliary. To determine the absolute configuration of the compound present, the pure diastereomeric salts and derivatives may be analysed with common spectroscopic methods, of which X-ray spectroscopy on single crystals constitutes a particularly suitable method.

Particular preference is given in each case to those compounds of the formula (I) in which at least one asymmetric carbon atom, for example one, two, three or preferably all four asymmetric carbon atoms, of the main chain have the stereochemistry (in each case "S") shown in the formula (IA) below, where the substituents are each as defined above, and their pharmaceutically usable salts:

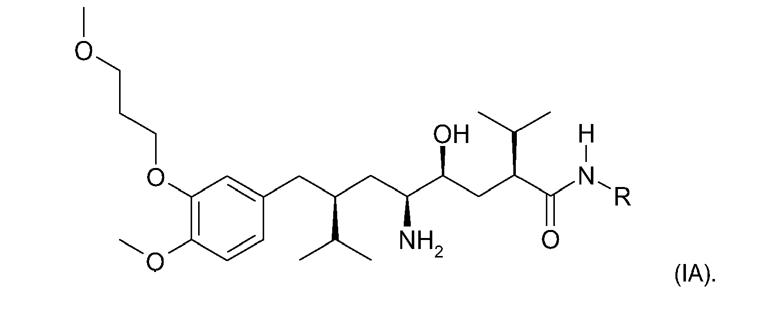

The compounds of the formula (I) or formula (IA) can be prepared in an analogous manner to literature preparation process (see WO 2002/008172 and WO 2002/002508 or literature cited there) (scheme).

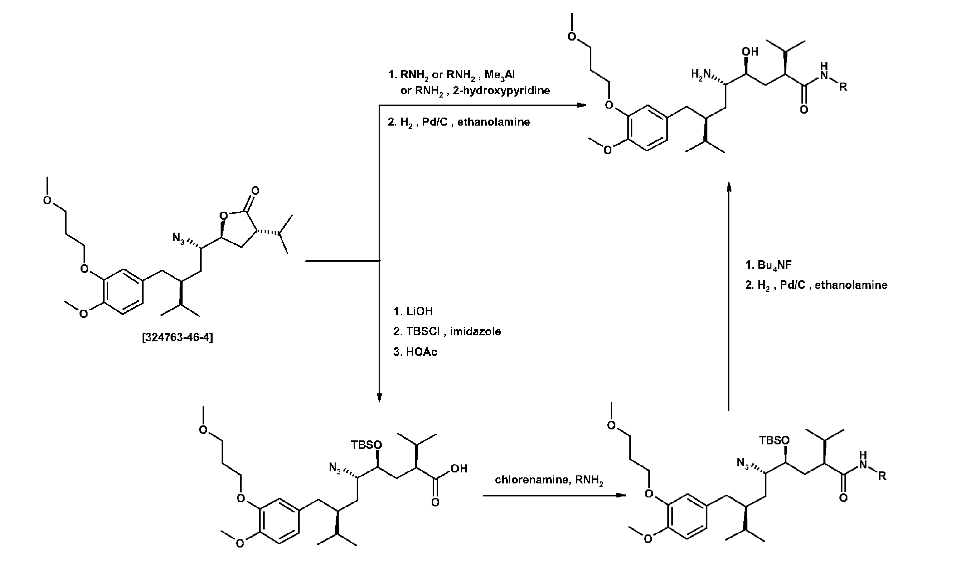

Details of the specific preparation variants can be taken from the examples.

The above-specified compound groups are not to be regarded as closed, but rather it is possible in a sensible manner, for example in order to replace general by more specific definitions, to exchange parts of these compound groups with one another or for the definitions given or to omit them.

The compounds of formula (I) and (IA), respectively, and their pharmaceutically useful salts reveal inhibitory activities on the enzymes beta-secretase, cathepsin D, plasmepsin II and/or HIV-protease.

The activitiy of inhibitors of beta-secretase, cathepsin D, plasmepsin II and/or HIV protease can be assessed experimentally with following *in vitro* assays.

The protease inhibitory activity of compounds can be tested with an assay kit using the fluorescence resonance energy transfer (FRET) technology and a recombinant i.e. baculovirus expressed enzyme preparation. The FRET is used to monitor the cleavage of the peptide substrate. The principle of the assay is as follows relies on a measurable energy difference, quantitatively depending on the presence of a peptide sequence. The peptide substrate is synthesized with two terminal fluorophores, a fluorescent donor and quenching acceptor. The distance between these two groups is selected so that upon light excitation, the donor fluorescence energy is significantly quenched by the acceptor through resonance energy transfer. Upon cleavage by the protease, the fluorophore is separated from the quenching group, restoring the fluorescence yield of the donor. Thus a weakly fluorescent peptide substrate becomes highly fluorescent upon enzymatic cleavage; the increase in fluorescence is linearly related to the rate of proteolysis.

The FRET assay was performed in white polysorp plates. The assay buffer consisted of 50 mM sodium acetate pH 5, 392 mM sodium chloride, 12.5% glycerol and 0.1 % BSA. The incubates per well were composed of 160 µl buffer, 10 µl inhibitor in DMSO, 10 µl peptide substrate in DMSO and 20 µl enzyme-solution. The inhibitors are tested in a concentration range of 1 pM to 1 mM. The fluorescently marked donor and acceptor peptide substrates are generated by solid phase peptide synthesis (Applied Biosystems). The beta-secretase peptide substrate Rh-Glu-Val-Asn-Leu-Asp-Ala-Glu-Phe-Lys-Quencher is obtained from Invitrogen, Carlsbad, CA, USA. The cathepsin D peptide substrate of the sequence DABCYL-Pro-Thr-Glu-Phe-Phe-Arg-Leu-OXL, the plasmepsin peptide substrate of the sequence DABCYL-Glu-Arg-Nle-Phe-Leu-Ser-Phe-Pro-OXL and the HIV protease peptide substrate of the sequence DABCYL-His-Lys-Ala-Arg-Val-Leu-Tyr-Glu-Ala-Nle-Ser-EDANS are all obtained from AnaSpec Inc, San Jose, CA, USA. The recombinantly expressed enzyme preparations are added in various amounts to the assay systems, e.g. the beta-sectrase concentration is 1 unit/ml incubation volume, the cathepsin D concentration is 100 ng/ml, the HIV protease concentration is 500 ng/ml and the plasmepsin II concentration is 50 ng/ml. The reaction is started upon addition of the enzyme solution. The incubation occurs at 37°C over 30-120 min ie specifically the beta-secretase incubation lasts 60 min, the cathepsin D incubation 120 min, the plasmepsin II incubation 40 min and the HIV protease incubation 40 min. The reactions are stopped by the addition of 20 µl of a 1.0 M Tris Base solution. The enzymatic substrate to product conversion is assessed by fluorescence measurements at 460 nm wave length.

In vitro enzyme inhibitory activities

The compounds of the present invention revealed structure-dependent and enzyme-specific inhibitory activities. The inhibitory activities were measured as IC50 values. Thus the beta-secretase inhibitory activity ranged between 1 pM and 1 mM; the values for cathepsin D ranged between 1 pM and 1 mM, for plasmepsin II between 1 pM and 1 mM and for HIV-protease between 1 pM and 1 mM.

The compounds of the formula (I) or preferred formula (IA) and the pharmaceutically usable salts thereof may find use as medicines, for example in the form of pharmaceutical preparations. The pharmaceutical preparations may be administered enterally, such as orally, for example in the form of tablets, coated tablets, sugar-coated tablets, hard and soft gelatine capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, coated tablets, sugar-coated tablets and hard gelatine capsules, the compounds of the formula (I) or preferred formula (IA) and pharmaceutically usable salts thereof may be processed with pharmaceutically inert, inorganic or organic excipients. Such excipients used, for example for tablets, coated tablets and hard gelatine capsules, may be lactose, corn starch, or derivatives thereof, talc, stearic acid or salts thereof etc.

Suitable excipients for soft gelatine capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical preparations may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

Subject of the present invention is also the use of the compounds of formula (I) and (IA), respectively, and their pharmaceutically useful salts for the prevention, delay of progression or the treatment of Alzheimer Disease, malaria or HIV infection.

Subject of the present invention is also the use of the compounds of formula (I) and (IA), respectively, and their pharmaceutically useful salts for the manufacture of a medication for the prevention, delay of progression or the treatment of Alzheimer Disease, malaria or HIV infection.

Subject of the present invention is also the method for the prevention, delay of progression or the treatment of Alzheimer Disease, malaria or HIV infection, whereby a therapeutically effective dose of a compound of the general formula (I) or preferred formula (IA) or a pharmaceutically usable salt thereof is applied.

Subject of the present invention is also a pharmaceutical preparation that contains for the inhibition of beta-secretase, cathepsin D, plasmepsin and/or HIV-protease a compound of the general formula (I), or preferred of formula (IA) or a pharmaceutically usable salt thereof as well as commonly used ingredients.

Subject of the present invention is also a pharmaceutical preparation for the prevention, delay of progression or treatment of Alzheimer Disease, malaria and HIV infection that contains a compound of the general formula (I), or preferred of formula (IA) or a pharmaceutically usable salt thereof as well as commonly used ingredients.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

### Examples

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is determined in the solvent system A. The ratio of solvents relative to one another is always reported in parts by volume. Chemical names for end products and intermediates were generated with the aid of the program AutoNom 2000 (automatic nomenclature).

HPLC gradients on Hypersil BDS C-18 (5 µm); column: 4 ×125 mm
I 90% water*/10% acetonitrile* to 0% water*/100% acetonitrile* in 5 minutes + 2.5 minutes (1.5 ml/min)
II 95% water*/5% acetonitrile* to 0% water*/100% acetonitrile* in 40 minutes (0.8 ml/min)
* containing 0.1 % trifluoroacetic acid

The following abbreviations are used:
- Rf: ratio of distance travelled by a substance to separation of the eluent front from the start point in thin-layer chromatography
- Rt: retention time of a substance in HPLC (in minutes)
- m.p.: melting point (temperature)

### General method A: (azide reduction)

A solution of 1 mmol of "azide derivative" in 10-20 ml of ethanol and ethanolamine (1 equiv.) is hydrogenated in the presence of 200-400 mg of 10% Pd/C (moist) at 0°C over 1-3 hours. The reaction mixture is clarified by filtration and the catalyst is washed with ethanol. The filtrate is concentrated by evaporation. The title compound is obtained from the residue by means of flash chromatography (SiO₂ 60F).

### General method B: (Lactone amidation I)

A mixture of 1 mmol of "lactone", "amine" (10-30 equiv.) and 2-hydroxypyridine (1 equiv.) is stirred at 65°C over 2-24 hours. The reaction mixture is cooled to room temperature, concentrated by evaporation, admixed with 1M aqueous sodium hydrogencarbonate solution and extracted with tert-butyl methyl ether (2x). The combined organic phases are washed with water and brine, dried over sodium sulphate and concentrated. The title compound is obtained from the residue by means of flash chromatography (SiO₂ 60F).

### General method C: (Lactone amidation II)

A solution of 1.1 mmol of trimethylaluminium solution (2 M in heptane) at -78°C is admixed with a solution of 1.2 mmol of "amine" in 1-2 ml of toluene. The reaction mixture is warmed to room temperature, stirred for a further 30-60 minutes and subsequently concentrated by evaporation. The residue is admixed with a solution of 1 mmol of "lactone" in 2 ml of toluene and stirred at 80°C for 2-4 hours. The reaction mixture is cooled to room temperature, admixed with 10 ml of 1 N HCl and then stirred for a further 30 minutes. The reaction mixture is diluted with brine and extracted with toluene (2x) - the combined organic phases are dried over sodium sulphate and concentrated by evaporation. The title compound is obtained from the residue by means of flash chromatography (SiO₂ 60F).

### General method D: (desilylation)

A solution of 1 mmol of "silyl ether" in 10-15 ml of tetrahydrofuran at 0°C is admixed with 1.5 mmol of tetrabutylammonium fluoride solution (1 M in tetrahydrofuran). The reaction mixture is stirred at room temperature over 2-4 hours, poured onto 1M aqueous sodium hydrogencarbonate solution and extracted with tert-butyl methyl ether (2x). The combined organic phases are washed with brine, dried over sodium sulphate and concentrated by evaporation. The title compound is obtained from the residue by means of flash chromatography (SiO₂ 60F).

### General method E: (Chlorenamine coupling)

A solution of 1 mmol of "acid" in 10 ml of dichloromethane at 0°C is admixed with 1.2-1.8 mmol of (1-chloro-2-methylpropenyl)dimethylamine(chlorenamine). After 2-4 hours, the reaction mixture is concentrated by evaporation and the residue is dissolved in 6 ml of dichloromethane - this solution is added dropwise to the solution of 1.25 mmol of "amine" and 1.1 mmol of triethylamine in 6 ml of dichloromethane at 0°C over 2-10 minutes. The reaction mixture is stirred at room temperature over 1-2 hours, poured onto water and extracted with tert-butyl methyl ether (2x). The combined organic phases are washed with brine, dried over sodium sulphate and concentrated by evaporation. The title compound is obtained from the residue by means of flash chromatography (SiO₂ 60F).

### General method F: (Lactone opening/silylation)

A solution of 1 mmol of "lactone" in 5 ml of dioxane is admixed with 5 ml of water and 1.1 mmol of lithium hydroxide monohydrate. After 4-6 hours, the reaction mixture is admixed with ice and 1M aqueous citric acid solution and extracted with tert-butyl methyl ether (3x). The combined organic phases are washed with cold water and cold brine, dried over sodium sulphate and concentrated by evaporation at room temperature. The residue is immediately dissolved in 8 ml of N,N-dimethylformamide and then admixed with 5 mmol of tert-butylchlorodimethylsilane and 8.8 mmol of imidazole. After 10-20 hours, the reaction mixture is concentrated by evaporation - the residue is admixed with diethyl ether and water, adjusted to pH 4 with 1 M aqueous citric acid solution, and then the organic phase is removed. The aqueous phase is extracted once more with diethyl ether (3x) - the combined organic phases are washed with brine, dried over sodium sulphate and concentrated by evaporation. The residue is dissolved in 3 ml of tetrahydrofuran and admixed successively with 3 ml of water and 9 ml of acetic acid. After 3-4 hours, the reaction mixture is poured onto ice-water and extracted with diethyl ether (2x) - the combined organic phases are washed with water and brine, dried over sodium sulphate and concentrated by evaporation. The title compound is obtained from the residue by means of flash chromatography (SiO₂ 60F).

### General method G: (N-Cbz protection)

A solution of 1 mmol of "amine" in 10 ml of ethyl acetate is admixed with 10 ml of saturated sodium carbonate solution and admixed at 0°C with 1.1 mmol of benzyl chloroformate. After 1-3 hours, the phases of the reaction mixture are separated. The aqueous phase is extracted with ethyl acetate (2x) - the combined organic phases are washed with 1M sodium hydrogencarbonate solution and brine, dried over sodium sulphate and concentrated by evaporation. The title compound is obtained from the residue by means of flash chromatography (SiO₂ 60F).

### General method H: (Pyridine oxidation)

A solution of 1 mmol of "pyridine" in 30 ml of dichloromethane is admixed with 2 mmol of m-chloroperbenzoic acid and stirred at room temperature for 2 hours. The mixture is poured onto 1M NaOH and extracted with tert-butyl methyl ether (3x). The combined organic phases are washed with 1M NaOH, water and brine, dried over sodium sulphate and concentrated by evaporation. The title compound is obtained from the residue by means of flash chromatography (SiO₂ 60F).

### General method I: (N-Cbz deprotection)

A solution of 1 mmol of "N-Cbz derivative" in 10-20 ml of ethanol and ethanolamine (1 equiv.) is hydrogenated in the presence of 200-400 mg of 10% Pd/C (moist) at 0°C over 1-3 hours. The reaction mixture is clarified by filtration and the catalyst is washed with ethanol. The filtrate is concentrated by evaporation. The title compound is obtained from the residue by means of flash chromatography (SiO₂ 60F).

### Example 1:

### N-(5-Chloropyridin-2-yl)-5(S)-amino-4(S)-hydroxy-2(S)-isopropyl-7(S)-[4-methoxy-3-(3-methoxypropoxy)benzyl]-8-methylnonamide

Analogously to method A, 0.088 g of N-(5-chloropyridin-2-yl)-5(S)-azido-4(S)-hydroxy-2(S)-isopropyl-7(S)-[4-methoxy-3-(3-methoxypropoxy)benzyl]-8-methyl-nonanamide is used to obtain the title compound as a colourless oil. Rf = 0.30 (200:20:1 dichloromethane-methanol-25% conc. ammonia); Rt = 4.51 (gradient I).

The starting material is prepared as follows:
a) N-(5-Chloropyridin-2-yl)-5(S)-azido-4(S)-hydroxy-2(S)-isopropyl-7(S)-[4-methoxy-3-(3-methoxypropoxy)benzyl]-8-methylnonanamide
   Analogously to method D, 0.11 g of N-(5-chloropyridin-2-yl)-5(S)-azido-4(S)-(tert-butyldimethylsilanyloxy)-2(S)-isopropyl-7(S)-[4-methoxy-3-(3-methoxypropoxy)benzyl]-8-methylnonanamide is reacted. The title compound is obtained as a colourless oil. Rf = 0.13 (1:2 EtOAc-heptane); Rt = 5.61 (gradient I).
b) N-(5-Chloropyridin-2-yl)-5(S)-azido-4(S)-(tert-butyldimethylsilanyloxy)-2(S)-isopropyl-7(S)-[4-methoxy-3-(3-methoxypropoxy)benzyl]-8-methylnonanamide
   Analogously to method E, 0.220 g of 5(S)-azido-4(S)-(tert-butyldimethylsilanyloxy)-2(S)-isopropyl-7(S)-[4-methoxy-3-(3-methoxypropoxy)benzyl]-8-methylnonanoic acid and 0.060 g of 5-chloropyridin-2-ylamine are reacted. The title compound is obtained as a colourless oil. Rf = 0.40 (1:2 EtOAc-heptane).
c) 5(S)-Azido-4(S)-(tert-butyldimethylsilanyloxy)-2(S)-isopropyl-7(S)-[4-methoxy-3-(3-methoxypropoxy)benzyl]-8-methylnonanoic acid
   Analogously to method F, 0.933 g of 5(S)-{1 (S)-azido-3(S)-[4-methoxy-3-(3-methoxypropoxy)benzyl]-4-methylpentyl}-3(S)-isopropyldihydrofuran-2-one [324763-46-4] is reacted. The title compound is obtained as a yellowish oil. Rf = 0.40 (1:1 EtOAc-heptane); Rt = 6.54 (gradient I).

### Example 2:

### N-(1-Oxypyridin-4-yl)-5(S)-amino-4(S)-hydroxy-2(S)-isopropyl-7(S)-[4-methoxy-3-(3-methoxypropoxy)benzyl]-8-methylnonanamide

Analogously to method I, 0.150 g of benzyl [(1S,2S,4S)-2-hydroxy-1-{(S)-2-[4-methoxy-3-(3-methoxypropoxy)benzyl]-3-methylbutyl}-5-methyl-4-(1-oxypyridin-4-ylcarbamoyl)hexyl]carbamate is used to obtain the title compound as a yellow foam. Rf = 0.53 (40:10:1 dichloromethane-methanol-25% conc. ammonia); Rt = 3.33 (gradient I).

The starting material is prepared as follows:
a) Benzyl [(1S,2S,4S)-2-hydroxy-1-{(S)-2-[4-methoxy-3-(3-methoxypropoxy)benzyl]-3-methylbutyl}-5-methyl-4-(1-oxypyridin-4-ylcarbamoyl)hexyl]carbamate
   Analogously to method H, 0.270 g of benzyl [(1S,2S,4S)-2-hydroxy-1-{(S)-2-[4-methoxy-3-(3-methoxypropoxy)benzyl]-3-methylbutyl}-5-methyl-4-(pyridin-4-ylcarbamoyl)hexyl]carbamate is used to obtain the title compound as a white foam. Rf = 0.13 (200:10:1 dichloromethane-methanol-25% conc. ammonia); Rt = 4.48 (gradient I).
b) Benzyl [(1S,2S,4S)-2-hydroxy-1-{(S)-2-[4-methoxy-3-(3-methoxypropoxy)benzyl]-3-methylbutyl}-5-methyl-4-(pyridin-4-ylcarbamoyl)hexyl]carbamate
   Analogously to method G, 0.231 g of N-(pyridine-4-yl)-(2S,4S,5S,7S)-5-amino-4-hydroxy-2-isopropyl-7-[4-methoxy-3-(3-methoxypropoxy)benzyl]-8-methylnon-anamide is used to obtain the title compound as a slightly yellowish foam. Rf = 0.45 (200:10:1 dichloromethane-methanol-25% conc. ammonia); Rt = 4.55 (gradient I).
c) N-(Pyridine-4-yi)-(2S,4S,5S,7S)-5-amino-4-hydroxy-2-isopropyl-7-[4-methoxy-3-(3-methoxypropoxy)benzyl]-8-methylnonanamide
   Analogously to method A, 0.231 g of N-(pyridine-4-yl)-(2S,4S,5S,7S)-5-azido-4-hydroxy-2-isopropyl-7-[4-methoxy-3-(3-methoxypropoxy)benzyl]-8-methylnon-anamide is used to obtain the title compound as a slightly yellowish foam. Rf = 0.16 (200:10:1 dichloromethane-methanol-25% conc. ammonia); Rt = 3.34 (gradient I).
d) N-(Pyridine-4-yl)-(2S,4S,5S,7S)-5-azido-4-hydroxy-2-isopropyl-7-[4-methoxy-3-(3-methoxypropoxy)benzyl]-8-methylnonanamide
   Analogously to method C, 0.4665 g of 5(S)-{1 (S)-azido-3(S)-[4-methoxy-3-(3-methoxypropoxy)benzyl]-4-methylpentyl}-3(S)-isopropyldihydrofuran-2-one [324763-46-4] is used to obtain the title compound as a white solid. Rf = 0.13 (95:5 dichloromethane-methanol); Rt = 4.54 (gradient I).

The processes described in examples 1 and 2 are used analogously to prepare the following compounds:

### Examples:

3 N-(4-Methylpyridin-2-yl)-5(S)-amino-4(S)-hydroxy-2(S)-isopropyl-7(S)-[4-methoxy-3-(3-methoxypropoxy)benzyl]-8-methylnonanamide
4 N-(5-Methylpyridin-2-yl)-5(S)-amino-4(S)-hydroxy-2(S)-isopropyl-7(S)-[4-methoxy-3-(3-methoxypropoxy)benzyl]-8-methylnonanamide
5 N-(3-Methylpyridin-2-yl)-5(S)-amino-4(S)-hydroxy-2(S)-isopropyl-7(S)-[4-methoxy-3-(3-methoxypropoxy)benzyl]-8-methylnonanamide
6 N-(6-Methylpyridin-3-yl)-5(S)-amino-4(S)-hydroxy-2(S)-isopropyl-7(S)-[4-methoxy-3-(3-methoxypropoxy)benzyl]-8-methylnonanamide
7 N-(2-Methylpyridin-4-yl)-5(S)-amino-4(S)-hydroxy-2(S)-isopropyl-7(S)-[4-methoxy-3-(3-methoxypropoxy)benzyl]-8-methylnonanamide
8 N-(6-Methylpyridin-2-yl)-5(S)-amino-4(S)-hydroxy-2(S)-isopropyl-7(S)-[4-methoxy-3-(3-methoxypropoxy)benzyl]-8-methylnonanamide
9 N-(5-Fluoropyridin-2-yl)-5(S)-amino-4(S)-hydroxy-2(S)-isopropyl-7(S)-[4-methoxy-3-(3-methoxypropoxy)benzyl]-8-methylnonanamide
10 N-(3,5-Difluoropyridin-2-yl)-5(S)-amino-4(S)-hydroxy-2(S)-isopropyl-7(S)-[4-methoxy-3-(3-methoxypropoxy)benzyl]-8-methylnonanamide
11 N-(5-Cyanopyridin-2-yl)-5(S)-amino-4(S)-hydroxy-2(S)-isopropyl-7(S)-[4-methoxy-3-(3-methoxypropoxy)benzyl]-8-methylnonanamide
12 N-(3-Fluoropyridin-2-yl)-5(S)-amino-4(S)-hydroxy-2(S)-isopropyl-7(S)-[4-methoxy-3-(3-methoxypropoxy)benzyl]-8-methylnonanamide
13 N-(6-Fluoropyridin-2-yl)-5(S)-amino-4(S)-hydroxy-2(S)-isopropyl-7(S)-[4-methoxy-3-(3-methoxypropoxy)benzyl]-8-methylnonanamide
14 N-(6-Trifluoromethylpyridin-3-yl)-5(S)-amino-4(S)-hydroxy-2(S)-isopropyl-7(S)-[4-methoxy-3-(3-methoxypropoxy)benzyl]-8-methylnonanamide
15 N-(2-Fluoropyridin-3-yl)-5(S)-amino-4(S)-hydroxy-2(S)-isopropyl-7(S)-[4-methoxy-3-(3-methoxypropoxy)benzyl]-8-methylnonanamide
16 N-(3-Fluoropyridin-4-yl)-5(S)-amino-4(S)-hydroxy-2(S)-isopropyl-7(S)-[4-methoxy-3-(3-methoxypropoxy)benzyl]-8-methylnonanamide
17 N-(2-Fluoropyridin-4-yl)-5(S)-amino-4(S)-hydroxy-2(S)-isopropyl-7(S)-[4-methoxy-3-(3-methoxypropoxy)benzyl]-8-methylnonanamide
18 N-(6-Fluoropyridin-3-yl)-5(S)-amino-4(S)-hydroxy-2(S)-isopropyl-7(S)-[4-methoxy-3-(3-methoxypropoxy)benzyl]-8-methylnonanamide
19 N-(1-Oxypyridin-2-yl)-5(S)-amino-4(S)-hydroxy-2(S)-isopropyl-7(S)-[4-methoxy-3-(3-methoxypropoxy)benzyl]-8-methylnonanamide
20 N-(1-Oxypyridin-3-yl)-5(S)-amino-4(S)-hydroxy-2(S)-isopropyl-7(S)-[4-methoxy-3-(3-methoxypropoxy)benzyl]-8-methylnonanamide

## Claims

1. Use of a compound of formula (I) 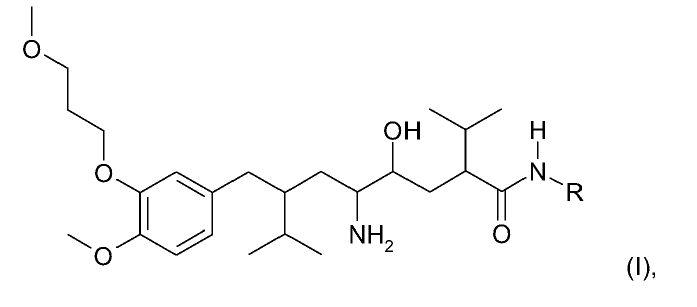 in which
R is heterocyclyl which is bonded via a carbon atom and is mono- or polysubstituted by C₁₋₆-alkyl, trifluoromethyl, nitro, amino, C₁₋₆-alkylamino, di-C₁₋₆-alkylamino, C₂₋₆-alkenyl, C₂₋₆-alkynyl, C₁₋₆-alkoxy, C₁₋₆-alkylcarbonyloxy, C₀₋₆-alkylcarbonylamino, C₁₋₆-alkoxycarbonylamino, hydroxyl, halogen, oxide, cyano, carbamoyl or N-mono- or N,N-di-C₁₋₈-alkylated carbamoyl, carboxyl or esterified carboxyl or C₁₋₆-alkylenedioxy, or is substituted by oxo and mono- or polysubstituted by C₁₋₆-alkyl, trifluoromethyl, nitro, amino, C₁₋₆-alkylamino, di-C₁₋₆-alkylamino, C₂₋₆-alkenyl, C₂₋₆-alkynyl, C₁₋₆-alkoxy, C₁₋₆-alkylcarbonyloxy, C₀₋₆-alkylcarbonylamino, C₁₋₆-alkoxycarbonylamino, hydroxyl, halogen, oxide, cyano, carbamoyl or N-mono- or N,N-di-C₁₋₈-alkylated carbamoyl, carboxyl or esterified carboxyl or C₁₋₆-alkylenedioxy,
or pharmaceutically usable salt or prodrug thereof, or where one or more atoms are replaced by their stable, non-radioactive isotopes for the manufacture of a medicament for the inhibition of beta-secretase, cathepsin D, plasmepsin II and/or HIV-protease.

2. Use according to claim 1 of a compound of formula (IA) or pharmaceutically usable salt 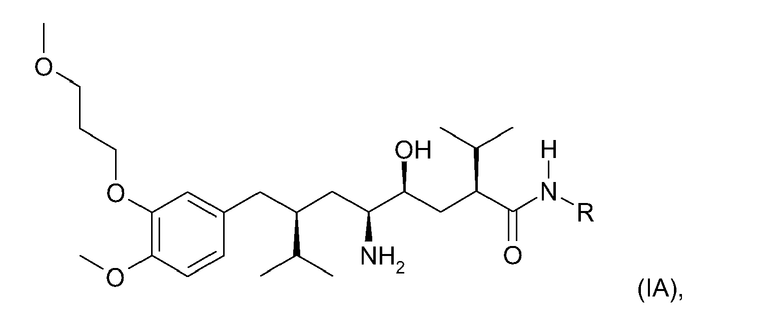 in which R is as defined for the compound of the formula (I).

3. Use according to one of claims 1 to 2, in which
R is a 5-10-membered mono- or bicyclic radical having at least one nitrogen or oxygen atom and optionally one oxygen or sulphur atom.

4. Use according to one of claims 1 to 3, in which
R is mono- or disubstituted whereby the substituents are selected from: C₁₋₆-alkyl, C₁₋₆-alkoxy, C₀₋₆-alkylcarbonylamino, C₁₋₆-alkoxycarbonylamino, trifluoromethyl, nitro, amino, hydroxyl, halogen, oxide, cyano, carbamoyl or N-mono- or N,N-di-C₁-C₈-alkylated carbamoyl and carboxyl or esterified carboxyl.

5. Use according to one of claims 1 to 4, in which
R is pyridyl, thiazolyl, oxazolyl, indolyl, benzofuranyl, benzothiazolyl or imidazolyl, each of which is bonded via a carbon atom and is substituted by C₁₋₆-alkyl, C₀₋₆-alkylcarbonylamino, C₁₋₆-alkoxycarbonylamino, C₁₋₆-alkoxy, trifluoromethyl, nitro, amino, hydroxyl, halogen, oxide, cyano, carbamoyl or carboxyl.

6. Use of a compound of the general formula (I) or (IA), or a pharmaceutically usable salt thereof, according to any one of claims 1 to 5 for the preparation of a medication for the prevention, delay of progression or treatment of Alzheimer Disease, malaria or HIV infection.

7. Pharmaceutical preparation for the prevention, delay of progression or treatment of Alzheimer disease, malaria or HIV infection containing a compound of the general formula (I) or (IA), or a pharmaceutically usable salt thereof, according to any one of claims 1 to 5 as well as commonly used ingredients.
